# EUROPEAN PATENT APPLICATION

(11) **EP 4 209 584 A1**
(43) Date of publication of application: **12.07.2023**
(21) Application number: 21864425.0
(22) Date of filing: 03.09.2021
(51) Int. Cl.: C12N 5/0775, A61K 35/28, A61P 43/00

(54) **PURIFIED CONCENTRATE OF CULTURE SUPERNATANT OF MESENCHYMAL STEM CELL OR PROGENITOR CELL DERIVED FROM SAID MESENCHYMAL STEM CELL, AND METHOD FOR PRODUCING SAME**

(30) Priority: 03.09.2020 JP 2020148193
(71) Applicant: Jichi Medical University, Tokyo 102-0093 (JP); Telebio K.K, Tokyo 150-0002 (JP)
(72) Inventor: YOSHIMURA, Kotaro, Tokyo 150-0012 (JP); SHIRADO, Takako, Shimotsuke-shi, Tochigi 329-0498 (JP); SAITO, Natsumi, Shimotsuke-shi, Tochigi 329-0498 (JP); WU, Yunyan, Shimotsuke-shi, Tochigi 329-0498 (JP)
(74) Representative: Godemeyer Blum Lenze Patentanwälte Partnerschaft mbB - werkpatent
(86) International application number: PCT/JP2021/032402
(87) International publication number: WO 2022/050373

(57) **Abstract**

It is an object of the present invention to provide a detoxified secretome extract of a culture supernatant having high efficacy and high safety, and a method for producing the same. According to the present invention, provided is a detoxified secretome extract of a culture supernatant of mesenchymal stem cells or progenitor cells derived from the mesenchymal stem cells, wherein the detoxified secretome extract comprises at least one of IGFBP, HGF, VEGF, PDGF, EGF, KGF (FGF-7), PDGFR, TGFα, and TGFβ secreted by the mesenchymal stem cells or the progenitor cells derived from the mesenchymal stem cells, and wherein lactic acid and ammonia that are metabolic waste products caused by the mesenchymal stem cells or the progenitor cells derived from the mesenchymal stem cells are removed from the detoxified secretome extract.

## Description

### Technical Field

The present invention relates to a detoxified secretome extract made from culture supernatant of mesenchymal stem cells or progenitor cells derived from the mesenchymal stem cells, which is useful for regenerative medicine. The present invention further relates to a method for producing the above-described detoxified secretome extract.

### Background Art

It has been known that cells release physiologically active substances for maintaining the normal functions of the cells themselves and cells that surround them, either in a living body or in a culture environment. In particular, in the case of somatic stem cells and/or progenitor cells, physiologically active substances specific to the somatic stem cells and/or the progenitor cells (e.g. cytokines, growth factors, angiogenic factors, exosomes, enzymes, extracellular matrixes, etc.) are released, and these physiologically active substances play important roles in regeneration of organs and the maintenance of homeostasis.

Stem cells are present in various sites in a whole body, such as bone marrow, fat, and skin. It has been reported that stem cells existing in subcutaneous adipose tissues (i.e. adipose-derived stromal/stem cells; ASCs) have almost the same properties as those of bone marrow-derived stem cells. When compared with the bone marrow-derived stromal/stem cell, the adipose-derived stromal/stem cells are advantageous in that the content of the cells in a tissue is high, in that a simple method of isolation from tissues, proliferation and preservation has been established, in that the amounts of various types of factors (e.g. a hepatocyte growth factor, a vascular endothelial cell growth factor, etc.) generated by the adipose-derived stromal/stem cells are large, in that the immunosuppressive ability of the adipose-derived stromal/stem cells is high, and in that less burden is imposed on a patient upon collection of the adipose-derived stromal/stem cells and after collection of the adipose-derived stromal/stem cells, the cell collection does not influence so much on the long-term health or lifespan of the patient.

With regard to the conventional treatment by administration of stem cells, it has been focused that the efficacy of humoral factors secreted by the stem cells has a larger impact than the survival and differentiation of the stem cells themselves and the functionality thereof. The use of a culture supernatant of stem cells as an injection, an external preparation or a cosmetic product has already started for the purpose of tissue repair, for cosmetic purpose, etc.

Patent Document 1 discloses a method for producing a preventive or a therapeutic agent for phacosclerosis, comprising a step of allowing mesenchymal stem cells to come into contact with the inner surface of a hollow fiber membrane, a step of refluxing a cell culture solution through the lumen and external cavity of the hollow fiber membrane, so as to culture the mesenchymal stem cells, and a step of recovering the cell culture solution refluxed through the lumen of the hollow fiber membrane.

Patent Document 2 discloses a cell culture device comprising: a culture vessel containing a cell suspension containing cells; a first filter portion having a first filter membrane that performs a membrane separation treatment on the cell suspension removed from the culture vessel; a first circulation channel that returns components blocked by the first filter membrane to the culture vessel; a second filter portion having a second filter membrane that performs a membrane separation treatment on components in the cell suspension that have permeated through the first filter membrane; a second circulation channel that returns components that have permeated through the second filter membrane to the culture vessel; and a recovery channel of recovering components blocked by the second filter membrane.

### Prior Art Documents

### Patent Documents

Patent Document 1: Patent Publication (Kokai) No. 2019-172607 A
Patent Document 2: International Publication WO2018/159847

### Summary of Invention

### Object to be Solved by the Invention

At present, the clinically utilized culture supernatant is a stock solution prepared by recovering a supernatant obtained after cell culture from a culture vessel and then removing stem cells mixed in the supernatant, as necessary, by centrifugal operations or filters, or a diluted solution of the stock solution. In such a culture supernatant, the concentrations of physiologically active substances such as a hepatocellular growth factor (HGF), insulin-like growth factors (IGFs), and a vascular endothelial growth factor (VEGF) are low, and metabolic waste products (lactic acid, ammonia, etc.) released from the cells are contained. Thus, the culture supernatant does not maximize its effectiveness. When a culture supernatant containing metabolic waste products is directly used in treatments, the culture supernatant is likely to cause damage on tissues or cells. It is an object of the present invention to provide a detoxified secretome extract from a culture supernatant having high efficacy and high safety, and a method for producing the same.

### Means for Solving the Object

The present inventions have conducted intensive studies directed towards achieving the aforementioned object. First, the present inventors have found that the amounts of physiologically active substances generated in a culture supernatant of mesenchymal stem cells or progenitor cells derived from the mesenchymal stem cells are influenced by an oxygen concentration, environmental stress, a cell density (confluency) upon cell culture, a culture period, the composition of a medium, a gravity, an atmospheric pressure, and the like. The inventors have focused on these culture conditions, and have established optimal culture protocols, so that the inventors have acquired a highly functional culture supernatant stock solution. Meanwhile, regarding harmful substances contained in the culture supernatant, the present inventors have confirmed that such harmful substances can be eliminated by a purification step performed by appropriate protocols. The present inventors have demonstrated that, by this detoxification step, concentration of useful physiologically active substances can be realized, as well as elimination of harmful substances. The present invention has been completed based on these findings.

According to the present invention, the following inventions are provided.
<1> A detoxified secretome extract of a culture supernatant of mesenchymal stem cells or progenitor cells derived from the mesenchymal stem cells, wherein
   the detoxified secretome extract comprises at least one of IGFBP, HGF, VEGF, PDGF, EGF, KGF (FGF-7), PDGFR, TGFα, and TGFβ secreted by the mesenchymal stem cells or the progenitor cells derived from the mesenchymal stem cells, and
   lactic acid and ammonia that are metabolic waste products caused by the mesenchymal stem cells or the progenitor cells derived from the mesenchymal stem cells are removed from the detoxified secretome extract.
<2> The detoxified secretome extract according to <1>, which is used as a therapeutic agent for regenerative medicine, an immunosuppressant, an anti-inflammatory agent, or an antifibrotic agent.
<3> The detoxified secretome extract according to <1> or <2>, which comprises all of IGFBP, HGF, VEGF, PDGF, EGF, KGF (FGF-7), PDGFR, TGFα, and TGFβ secreted by the mesenchymal stem cells or the progenitor cells derived from the mesenchymal stem cells.
<4> The detoxified secretome extract according to any one of <1> to <3>, wherein at least one of IGFBP, HGF, VEGF, PDGF, EGF, KGF (FGF-7), PDGFR, TGFα, and TGFβ is 1.2 times or more concentrated, compared with the culture supernatant before concentration.
<5> The detoxified secretome extract according to any one of <1> to <4>, wherein the mesenchymal stem cells or the progenitor cells derived from the mesenchymal stem cells are adipose-derived stromal/stem cells or adipose-derived vascular endothelial progenitor cells.
<6> The detoxified secretome extract according to any one of <1> to <5>, wherein the ammonia concentration is 20 µg/dL or less, and the HGF concentration is 50000 pg/mL or more.
<7> A method for producing the detoxified secretome extract according to any one of <1> to <6>, comprising:
   a culture supernatant-obtaining step of obtaining a culture supernatant by culturing mesenchymal stem cells or progenitor cells derived from the mesenchymal stem cells in a culture solution;
   a concentration step of concentrating an active ingredient from the culture supernatant; and
   a purification step of removing metabolic waste products from the concentrated culture supernatant.
<8> The method according to <7>, wherein, in the culture supernatant-obtaining step, a culture supernatant is obtained from a culture of the mesenchymal stem cells that are in a proliferation phase or a confluent phase, or the progenitor cells derived from the mesenchymal stem cells.
<9> The method according to <7> or <8>, wherein the metabolic waste products removed in the purification step are lactic acid and ammonia.
<10> The method according to any one of <7> to <9>, wherein the purification step is carried out by ultrafiltration.
<11> The method according to <10>, wherein the ultrafiltration is carried out using a filtration membrane having a cutoff molecular weight of 2 kDa to 30 kDa.

### Advantageous Effects of Invention

According to the method of the present invention, when compared with a culture supernatant recovered by the conventional recovery method, the proliferative ability of cells cultured *ex vivo* can be increased by about two times. That is to say, according to the present invention, a culture supernatant, which is safe and has a high regenerative medicine effect, can be supplied. Since the detoxified secretome extract of the culture supernatant of the present invention can be produced in a large amount and does not contain cells, it can also be utilized as a product from allogeneic cells.

### Brief Description of Drawings

[Fig. 1] Fig. 1 shows the influence of inhibitory substances on ASC cell proliferation activity (Mean ± SD, n = 4, WST8 assay).
[Fig. 2] Fig. 2 shows the effect of removing lactic acid from CM using an ultrafiltration membrane (Mean ± SD, n = 3).
[Fig. 3] Fig. 3 shows the results obtained by studying oxygen culture conditions for accelerating a cell proliferation speed.
[Fig. 4] Fig. 4 shows an ASC-seeding density and the amount of HGF secreted in a culture supernatant (n = 1).
[Fig. 5] Fig. 5 shows the confluency of ASC and the amount of HGF.
[Fig. 6] Fig. 6 shows the types of growth factors secreted into CM.
[Fig. 7] Fig. 7 shows the recovery of a culture supernatant and the schedule of medium exchange in Example 5.
[Fig. 8] Fig. 8 shows the results obtained by measuring the influence of the oxygen concentration and the presence or absence of serum (10% FBS) on HGF production.
[Fig. 9] Fig. 9 shows the results obtained by measuring HGF in a culture supernatant, a concentrate, and a waste liquid after concentration.
[Fig. 10] Fig. 10 shows the results obtained by measuring the ammonia concentration in a culture supernatant, a concentrate, and a waste liquid after concentration.
[Fig. 11] Fig. 11 shows the results obtained by measuring the influence of a culture supernatant, a concentrate, and a waste liquid after concentration on the proliferative ability of hASCs (cell proliferation experiment).
[Fig. 12] Fig. 12 shows the results obtained by measuring the influence of a culture supernatant and a detoxified secretome extract on the proliferation of human dermal fibroblasts and normal human epidermal keratinocytes (cell proliferation experiment).
[Fig. 13] Fig. 13 shows the results obtained by measuring the influence of a culture supernatant and a culture supernatant detoxified secretome extract on the tube-forming ability of vascular endothelial cells (endothelial cell tube formation test).
[Fig. 14] Fig. 14 shows the results obtained by evaluating the effect of promoting wound healing in type 2 diabetic mice.
[Fig. 15] Fig. 15 shows the results obtained by measuring the influence of a platelet lysate on the cell proliferative ability and HGF-producing ability of ASC.
[Fig. 16] Fig. 16 shows the results obtained by measuring the influence of various types of growth factors on the HGF-producing ability of ASC.
[Fig. 17] Fig. 17 shows the results obtained by measuring the influence of a culture supernatant and a detoxified secretome extract on inflammatory cytokine production.

### Embodiments of Carrying out the Invention

Hereinafter, the embodiments of the present invention will be specifically described.

### [1] Detoxified secretome extract of culture supernatant of mesenchymal stem cells or progenitor cells derived from mesenchymal stem cells

The present invention relates to a detoxified secretome extract obtained by recovering a culture supernatant of mesenchymal stem cells or progenitor cells derived from the mesenchymal stem cells, which have been cultured *ex vivo,* and then purifying and concentrating the recovered culture supernatant. Such a culture supernatant of mesenchymal stem cells or progenitor cells derived from the mesenchymal stem cells contains growth factors secreted from the cells, and physiologically active substances such as cytokines, exosomes, angiogenic factors and enzymes. These physiologically active substances play important roles in the functional recovery of cells injured by damage and/or aging, or regeneration of tissues and/or organs, and the physiologically active substances can be utilized as regenerative medicines in the treatment of various refractory diseases.

Specifically, the present invention relates to a detoxified secretome extract of a culture supernatant of mesenchymal stem cells or progenitor cells derived from the mesenchymal stem cells, wherein the detoxified secretome extract comprises at least one of IGFBP (IGFBP-1, IGFBP-2, IGFBP-3, IGFBP-4, IGFBP-6, etc.), HGF, VEGF (VEGF-A, etc.), PDGF (PDGF-AA, PDGF-AB, PDGF-BB, etc.), EGF, KGF (FGF-7), PDGFR (PDGFR-α, PDGFR-β, etc.), TGFα, and TGFβ secreted by the mesenchymal stem cells or the progenitor cells derived from the mesenchymal stem cells, and wherein lactic acid and ammonia that are metabolic waste products caused by the mesenchymal stem cells or the progenitor cells derived from the mesenchymal stem cells are removed from the detoxified secretome extract.

The detoxified secretome extract of the present invention may comprise at least one of IGFBP, HGF, VEGF, PDGF, EGF, KGF (FGF-7), PDGFR, TGFα, and TGFβ. The present detoxified secretome extract preferably comprises at least two of the aforementioned substances, and more preferably comprises all of the aforementioned substances.

In the detoxified secretome extract of the present invention, at least one of IGFBP, HGF, VEGF, PDGF, EGF, KGF (FGF-7), PDGFR, TGFα, and TGFβ (preferably at least two of the aforementioned substances, and more preferably all of the aforementioned substances) is preferably 1.2 times or more concentrated, compared with the culture supernatant before concentration. The concentration magnification may also be 1.5 times or more, 2 times or more, 3 times or more, 5 times or more, 10 times or more, 15 times or more, 20 times or more, 25 times or more, or 30 times or more.

In the detoxified secretome extract of the present invention, the ammonia concentration is preferably 20 µg/dL or less, more preferably 15 µg/dL or less, and further preferably 10 µg/dL or less. The HGF concentration is preferably 50000 pg/mL or more, and more preferably 50,000 pg/mL or more and 2500,000 pg/mL or less. The HGF concentration may also be 100,000 pg/mL or more, or 200,000 pg/mL or more.

Examples of mesenchymal stem cells or progenitor cells derived from the mesenchymal stem cells may include adipose-derived stem cells, bone marrow-derived stem cells, cord blood-derived stem cells, and progenitor cells derived from those cells.

The adipose-derived stem cells may be, for example, vascular endothelial (progenitor) cells.

The adipose-derived stem cells are adipose-derived cells having pluripotency. The adipose-derived stem cells are capable of differentiating into adipocytes, osteoblasts, chondroblasts, myofibroblasts, osteocytes, muscle cells, nerve sheath cells, or the like. The ratio of such adipose-derived stem cells can be confirmed as CD31-negative and CD90-positive cells. The adipose-derived stem cells can also be confirmed as CD45-negative, CD44-positive, CD29-positive, and CD13-positive cells. The above-described markers can be measured by FACS (fluorescence activated cell sorting).

The vascular endothelial (progenitor) cells are cells that constitute the inner surface of a blood vessel and are contacted with a lumen in which the blood circulates. The vascular endothelial (progenitor) cells have a concept including both vascular endothelial cells and vascular endothelial progenitor cells. The vascular endothelial (progenitor) cells retain an ability to divide and proliferate. The vascular endothelial (progenitor) cells can be identified using the property of being negative to CD45 and positive to CD31 as an indicator. The vascular endothelial (progenitor) cells can also be identified as CD146-positive and CD144-positive cells.

### [2] Method for producing detoxified secretome extract of culture supernatant of mesenchymal stem cells or progenitor cells derived from mesenchymal stem cells

The present invention relates to a method for producing a detoxified secretome extract of a culture supernatant of mesenchymal stem cells or progenitor cells derived from the mesenchymal stem cells, wherein the method comprises: a culture supernatant-obtaining step of obtaining a culture supernatant by culturing mesenchymal stem cells or progenitor cells derived from the mesenchymal stem cells in a culture solution; a concentration step of concentrating the above-described culture supernatant; and further, a purification step of removing metabolic waste products from the concentrated culture supernatant.

Adipose-derived stem cells or progenitor cells derived from the adipose-derived stem cells can be obtained from adipose tissues. The adipose tissues can be obtained, for example, from humans, or from mammals other than the humans, birds, or the like, by surgical resection. Examples of the mammals other than human may include dogs, cats, bovines, horses, pigs, goats, sheep, monkeys, ferrets, rabbits, mice, rats, gerbils, Guinea pigs, and hamsters. The birds may be, for example, chickens. Upon performing surgical resection, the animals may be undergone local anesthesia. The adipose tissues can also be obtained by suction by inserting a cannula into the subcutaneous adipose tissues of the abdomen, thighs, buttocks, or whole body. The amount of the obtained adipose tissues is for example, 0.1 g to 1000 g, and preferably 1 g to 500 g, 1g to 100 g, 2 g to 50 g, or 2 g to 40 g, but it is not limited thereto.

The obtained adipose tissues are preferably confirmed with naked eyes, in terms of the absence of tumoral lesion or contamination. It may also be adequate if the adipose tissues are confirmed to be tested negative to all of HBV, HCV, HIV, HTLV-1, and TPHA/RPR. It may also be confirmed that mycoplasma is less than 128 times (PA method) and herpes simplex is less than 320 times (CF method) in the adipose tissues.

Adipose-derived stem cells can be prepared by a known method, and the method of preparing adipose-derived stem cells is not particularly limited. For example, suctioned adipose tissues are placed at rest, and an oil layer, an adipose layer and a water layer are then separated from the adipose tissues. Thereafter, the oil layer and the water layer are discarded, and only the adipose layer can be recovered. Subsequently, the obtained adipose layer can be subjected to an enzyme treatment. Otherwise, it is also possible to allow fragmented adipose tissues to directly adhere onto a culture dish or the like, and then to promote proliferation of adipose-derived cells and an adhesion culture thereof.

The adipose tissues before being subjected to an enzyme treatment are preferably warmed in a water bath at room temperature or at 37°C for 5 minutes to 15 minutes, before the enzyme treatment.

The enzyme treatment can be carried out by adding an appropriate amount of enzyme reaction solution into an adipose layer in a tube, fixing the tube in a constant temperature shaker, and then shaking the tube. The temperature for the enzyme treatment is not particularly limited, as long as the enzyme reaction progresses. The enzyme treatment temperature is generally 25°C to 50°C, and preferably 30°C to 45°C. The enzyme treatment temperature is, for example, 37°C. The shaking applied herein may be either reciprocating shaking or orbital shaking. In the case of reciprocating shaking, the reciprocating shaking rate is not particularly limited, and it is generally 10 rpm to 300 rpm, and preferably 50 rpm to 200 rpm. The reciprocating shaking rate is, for example, 120 rpm. The reaction time is not particularly limited, and it is generally 10 minutes to 3 hours, and preferably 15 minutes to 1 hour. The reaction time is, for example, 30 minutes.

As an enzyme, at least collagenase is preferably used.

Collagenase is an enzyme generated by animal tissue cells, inflammatory cells, tumor cells, or bacteria such as *Clostridium histolyticum,* or a recombinant protein artificially produced by genetic recombination techniques; and the collagenase is an enzyme that decomposes type I, type II, and type III collagens. Further, neutral protease, thermolysin, trypsin, dispase or the like may also be added.

The final concentration of collagenase in the enzyme treatment solution is preferably 0.02% to 2%, more preferably 0.1% to 1%, even more preferably 0.1% to 0.5%, further preferably 0.1% to 0.4%, still further preferably 0.1% to 0.3%, and most preferably 0.2%.

It is preferable that the enzyme treatment solution used in the enzyme treatment further comprises DNaseI, as well as collagenase. In the case of using DNaseI, the concentration of DNaseI in the enzyme treatment solution is preferably 100 to 10000 U/mL, more preferably 200 to 5000 U/mL, and further preferably 500 to 2000 U/mL.

It is preferable that the enzyme treatment solution used in the enzyme treatment further comprises CaCh. The concentration of CaCh in the enzyme treatment solution is preferably 0.01 mM to 10 mM, more preferably 0.1 mM to 5 mM, and further preferably 1 mM to 4 mM. The concentration of CaCh in the enzyme treatment solution is, for example, 3 mM.

The enzyme treatment solution used in the enzyme treatment is preferably a buffer solution, and is more preferably HBSS (Hanks' Balanced Salt Solution).

A mixture of an adipose layer and an enzyme solution is rotated in a centrifuge tube at a temperature suitable for the enzyme reaction (for example, 30°C to 45°C, and preferably 35°C to 40°C), so that the mixture can be allowed to react. After completion of the reaction, the reaction mixture was further centrifuged at 800 G, so that it can be separated into 3 layers, namely, an oil layer, an adipose layer, a water layer, and a precipitate layer (cell layer) from above. By removing the oil layer, the adipose layer and the water layer, only the precipitate layer (cell layer) can be obtained. The precipitate layer is suspended in a suitable normal saline, so that a suspension of adipose-derived cells (stromal vascular fraction (SVF)) can be obtained.

In the present invention, mesenchymal stem cells or progenitor cells derived from the mesenchymal stem cells are cultured in a culture solution to obtain a culture supernatant.

The culture solution (medium) is not particularly limited, as long as it is capable of culturing mesenchymal stem cells or progenitor cells derived from the mesenchymal stem cells. Examples of the medium used herein may include EGM-2 (Lonza), αMEM, Dulbecco's Modified Eagle Medium (DMEM), Dulbecco's Modified Eagle Medium/Nutrient Mixture F-12 Ham (DMEM/F12), and RPMI1640. In general, to these culture solutions, various types of additives applicable to an ordinary cell culture, such as serum, various types of vitamins, various types of antibiotics, various types of hormones, and various types of growth factors, may be added. As a medium, DMEM/F12, EGM-2 or EGM-2MV (both of which are manufactured by Lonza), or the like can be particularly preferably used.

The culture is preferably carried out using a culture vessel such as a flask in 5% CO₂ at 37°C. The medium may be exchanged with a fresh one, for example, every two days. The culture can be carried out at an oxygen concentration of 1% to 21%. The culture is particularly preferably carried out at an oxygen concentration of 2% to 6%.

The culture period is not particularly limited, and the culture is carried out, for example, for 1 day to 14 days. After the culture has been carried out for 1 day to 6 days, the cells may be passaged and may be cultured again, for example, for 3 days to 6 days. The number of passages and the number of cultures are not particularly limited.

The seeding of the culture suspension is not particularly limited, and as an example, the seeding is carried out at a density of 5.1 × 10⁴ viable nucleated cells/cm² and in a medium amount of about 0.25 mL/cm². After a stage in which the passage culture can be stably carried out, the seeding can be carried out, for example, at a density of 4000 viable nucleated cells/cm² and in a medium amount of 0.25 mL/cm².

In the culture supernatant-obtaining step, a culture supernatant can be obtained from a culture of mesenchymal stem cells or progenitor cells derived from the mesenchymal stem cells that are in a proliferation phase or a confluent phase.

The culture supernatant can be prepared by a method known in the present technical field. For example, the obtained culture solution is centrifuged and is passed through a filter (or a strainer) having an appropriate pore diameter, so as to obtain a culture supernatant. Herein, the centrifugation can be carried out, for example, at 300 to 1200 x g for 5 to 20 minutes.

In the present invention, a concentration step of concentrating useful substances contained in the culture supernatant, and further, a purification step of removing metabolic waste products from the concentrated culture supernatant are carried out. The concentration step can be carried out by a method known in the present technical field. Examples of the concentration method may include ultrafiltration, gel filtration, and dialysis, but the examples are not limited thereto. In the purification step, the diluted concentrated culture supernatant is further subjected to a similar filtration step, so that lactic acid and ammonia that are metabolic waste products can be reduced or removed. Thus, by reducing the metabolic waste products to certain concentrations or less, the culture supernatant can be detoxified.

The concentration and purification steps can be preferably carried out by ultrafiltration.

When these steps are carried out by an ultrafiltration method, the ultrafiltration is preferably carried out using a filtration membrane having a cutoff molecular weight of 2 kDa to 30 kDa. The cutoff molecular weight of the filtration membrane may also be 2 kDa to 20 kDa, 2 kDa to 10 kDa, 2 kDa to 5 kDa, or 2 kDa to 4 kDa.

The concentration magnification of the culture supernatant is preferably 1 to 100 times, and more preferably 20 to 50 times, when the culture supernatant is in a liquid state upon transplantation. As a result of the concentration, a greater healing-promoting effect or immunosuppressive effect can be expected. By repeating the concentration step, it is also possible to further concentrate the culture supernatant without limits.

The present invention is characterized in that the concentration and the purification are each carried out in a different step. A culture supernatant is recovered from a flask, and cells mixed in the culture supernatant are removed by centrifugation and a filter (for example, a 0.22 µm PES sterilization filter), so as to obtain a cell-removed culture supernatant. The cell-removed culture supernatant is concentrated by passing through an ultrafiltration membrane, so that the concentrated cell-removed culture supernatant (which is referred to as "CCM-pre" in the Examples) is recovered in the ultrafiltration filter (concentration step). Subsequently, a fresh basal medium was added to the ultrafiltration filter to the maximum loading capacity, and ultrafiltration is performed again (purification step). Thereby, in the case of a concentrate having a low molecular weight, the concentrate is almost replaced with the components of the basal medium (that is, low-molecular-weight metabolites such as ammonia and lactic acid are eliminated by being replaced with such a fresh basal medium), and thus, a detoxified secretome extractd cell-removed culture supernatant can be obtained. In the present invention, both the concentration step and the purification step may be repeatedly carried out multiple times. In addition, the medium can be replaced with any given solvent (e.g. PBS, Ringer's solution, distilled water, etc.), as appropriate.

The thus obtained culture supernatant detoxified secretome extract does not comprise cells, but comprises at least one of IGFBP, HGF, VEGF, PDGF, EGF, KGF (FGF-7), PDGFR, TGFα, and TGFβ secreted by mesenchymal stem cells or progenitor cells derived from the mesenchymal stem cells, and lactic acid and ammonia that are metabolic waste products caused by the mesenchymal stem cells or the progenitor cells derived from the mesenchymal stem cells are removed from the detoxified secretome extract.

### [3] Composition for treatment or beauty

The detoxified secretome extract of the present invention can be used as a composition for the purpose of the treatment of the damage, degeneration, disorder or dysfunction of biological tissues or organs, or beauty. That is to say, the detoxified secretome extract of the present invention can be provided as a pharmaceutical composition for a therapeutic agent for regenerative medicine, an immunosuppressant, an anti-inflammatory agent, an antifibrotic agent or the like, or as a beauty composition. Specifically, the effects of angiogenesis, tissue regeneration, and immunosuppression can be obtained. Hence, regarding the skin, beauty or rejuvenation effects for the purpose of improving wrinkles and/or stains, or therapeutic effects on ED and thinning hair can be expected. The immunosuppressive effects possessed by this composition can be broadly applied to the treatment of allergic diseases including atopic dermatitis as a typical example, the treatment of various autoimmune diseases including rheumatoid arthritis as a typical example, the treatment of cytokine storm found in severe new coronavirus, or the like.

By transplantation of a culture supernatant detoxified secretome extract that does not contain cells, immune rejection attended with cell transplantation and the risk of GVHD can be avoided, and operations performed upon administration can be simplified. In addition, such a culture supernatant detoxified secretome extract can be preserved, and it can be cryopreserved in the state of a liquid, or it can be preserved for a long period of time in the formed of a dry powder product as a result of freeze-drying, without being deteriorated. Moreover, since autogenous transplantation is desirable as such cell transplantation, cells to be transplanted must be collected from a healthy site by an invasive method. In contrast, the transplantation of a culture supernatant detoxified secretome extract does not need it. Furthermore, in the transplantation of a culture supernatant detoxified secretome extract, administration of a large dose is possible. Further, 2 or 3 types of purified and concentrated culture supernatants (for example, an adipose stem cell-derived culture supernatant and a vascular endothelial cell-derived culture supernatant) can be mixed with one another, and the obtained mixture can be then used. Further, PRP (platelet-rich plasma) that has been reported to have therapeutic effects when it is administered for intractable cutaneous ulcer can be mixed with the culture supernatant detoxified secretome extract, and the obtained mixture can be then used.

By using the detoxified secretome extract of the present invention in combination with stem cells, various organs or tissues can be efficiently regenerated, and the detoxified secretome extract of the present invention and the stem cells can be engrafted in the transplanted site. In particular, angiogenesis and regeneration or remodeling of tissues can be promoted.

By combining the detoxified secretome extract of the present invention with adipose tissues, with an adipose tissue-derived stromal vascular fraction (SVF), or with adipose stem cells or vascular endothelial (progenitor) cells, the present detoxified secretome extract can be used in transplantation for the purpose of breast augmentation, breast reconstruction after breast cancer resection, the improvement of wrinkles around eyes and cheeks or the improvement of the declined skin resilience, etc.

The detoxified secretome extract of the present invention is used in combination with osteoblasts, so that transplantation can be carried out for the purpose of the treatment of bone fracture, bone lengthening for improving short stature, bone deformation or leg length discrepancy, etc.

The detoxified secretome extract of the present invention is used in combination with chondrocytes, so that transplantation can be carried out for the purpose of treating diseases associated with retrogression, degeneration, deformation and other abnormities of the articular cartilage (for example, osteoarthritis, rheumatoid arthritis, shoulder periarthritis, temporomandibular disorder, etc.).

The detoxified secretome extract of the present invention is used in combination with smooth muscle cells, so that transplantation can be carried out for the purpose of treating diseases caused by the damage or abnormity of smooth muscle cells (for example, dysuria (incontinence, frequent urination, urinary retention, etc.), leiomyoma, leiomyosarcoma, etc.).

The composition of the present invention may be diluted with an infusion formulation, distilled water for injection, or a culture solution, which is used as a pharmaceutically acceptable medium. The infusion formulation is not particularly limited, and examples thereof may include a normal saline, a 5% glucose solution, a Ringer's solution, a lactated Ringer's solution, an acetated Ringer's solution, an initiation solution (No. 1 solution), a rehydration solution (No. 2 solution), a maintenance transfusion (No. 3 solution), and a postoperative recovery solution (No. 4 solution). Upon dilution, the number of cells is not particularly limited, and it can be set to be, for example, 1 × 10³ to 1 × 10⁷ cells/mL.

The composition of the present invention may comprise various additives for increasing preservation stability, sterility, isotonicity, absorbency and/or viscosity, such as, for example, an emulsifier, a dispersant, a buffer, a preservative, a wetting agent, an antibacterial agent, an antioxidant, a chelating agent, a thickener, a gelling agent, and a pH adjuster. Examples of the thickener may include HES, dextran, methyl cellulose, xanthan gum, carboxymethyl cellulose, and hydroxypropyl cellulose, but the examples are not limited thereto.

The applied dose of the composition of the present invention can be determined, as appropriate, depending on the dosage form, the administration method, the use purpose, the age, body weight and symptoms of a patient or a subject, etc.

The applied dose of the detoxified secretome extract for a single administration is not particularly limited. For example, if the applied dose of the detoxified secretome extract is expressed with the volume of a culture supernatant before purification and concentration used as a raw material, it is 0.01 mL/kg body weight or more, 0.1 mL/kg body weight or more, or 1 mL/kg body weight or more. On the other hand, the single dose of the detoxified secretome extract is not particularly limited, and it is, for example, 10 mL/kg body weight or less, or 5 mL/kg body weight or less.

The administration method of the composition of the present invention is not particularly limited. Examples of the administration method applied herein may include subcutaneous injection, intralymph node injection, intravenous injection, intraarterial injection, intraperitoneal injection, intrapleural injection, and direct injection into a local site. Otherwise, direct transplantation into a local site with the use of a catheter may also be applied. Furthermore, the composition of the present invention can also be administered by external application as an ointment or an emulsion, or by spray coating as a lotion, or also in the form of an internal medicine.

The subjects, to which the composition of the present invention is administered, are typically humans, but the subjects may also be other animals. Examples of such other animals may include mammals and birds. Examples of the mammals may include dogs, cats, bovines, horses, pigs, goats, sheep, monkeys, ferrets, rabbits, mice, rats, gerbils, Guinea pigs, and hamsters. The birds may be, for example, chickens.

The present invention will be specifically described in the following examples. These examples are not intended to limit the scope of the present invention.

### Examples

### Components to be prepared

#1: HBSS without Ca++, without Mg++ (Gibco, #14175-095)
#2: Roughly purified collagenase derived from *Clostridium histolyticum* (FUJIFILM Wako Pure Chemical Corporation, #032-22364)
#3: Roughly purified DNase1 (Worthington, #LS002139)
#4: Centrifuge tube (CORNING, #431123)
#5: Swing-type desktop centrifuge (KUBOTA, #S700T)
#6: Constant temperature shaker (Yamato Scientific Co., Ltd. , #BT100)
#7: Electronic scales
#8: Dropper silicon rubber (AS ONE Corporation, #6-356-04)
#9: Cell strainer (CORNING, 100 µm; #352360, 40 µm; #3552340)
#10: Cell counter (Logos Biosystems, #L30001)
#11: Calcium chloride (FUJIFILM Wako Pure Chemical Corporation, #037-24031)
#12: Syringe filter with diameter of 0.22 µm (Millipore, #2LGV-33RS)
#13: CELLBANKER 1 (Takara Bio, Inc., #CD011)
#14: Cell culture dish (CORNING, #353025)
#15: DMEM/Ham's F-12 (FUJIFILM Wako Pure Chemical Corporation, #048-29785) #16: Fetal Bovine Serum (FBS)
#17: Penicillin-streptomycin (100x) (FUJIFILM Wako Pure Chemical Corporation, #168-23191)
#18: CO₂ incubator (Panasonic, #MCO-170AICUVH-PJ)
#19: TrypLE Express (Gibco, #12604-021)
#20: L-(+)-lactic acid (Sigma-Aldrich, #L6402)
#21: 10% ammonia water (FUJIFILM Wako Pure Chemical Corporation, #013-17505)
#22: 96-Well plate (PerkinElmer, SpectraPlate-96, #6005650)
#23: Cell Counting Kit-8 (Dojindo, #CK04)
#24: Plate reader (PerkinElmer, ARVO MX)
#25: Ultrafiltration membrane (Millipore, Amicon Ultra-15 10 kDa, #UFC901024)
#26: Lactic acid quantification kit (Abbott, GC4+)
#27: Hypoxic CO₂ incubator (WakenBtech Co., Ltd.)
#28: Human HGF ELISA kit (R & D systems, Quantikine 96-well plate #DHG00B)
#29: Growth factor antibody array (Raybio, #AAH-GF-1)
#30: Lumino Image analyzer (FUJIFILM Corporation, LAS-3000mini)

^{∗}1: Preparation of 1 M calcium chloride solution
   11.1 g of Calcium chloride (#11) was diluted with 100 mL of Milli Q water, and was then subjected to sterilization in an autoclave (121°C, 20 minutes).
*2: Preparation of 1 × 10⁵ units/mL DNase1 solution
   DNase1 (#2) was dissolved in Milli Q water to a concentration of 1 × 10⁵ units/mL.
   The obtained solution was sterilized through a syringe filter (#12), was then dispensed in a sterilization tube, and was then stocked at -30°C.
*3: Preparation of enzyme reaction solution
   Solution A
      0.2% (w/v) Collagenase (#2), 0.264g
      3 mM Calcium chloride [1 M stock solution (*1)], 0.396mL
      HBSS (#1), 65.6 mL
      The solution was subjected to filtration sterilization using a filter unit such as a syringe filter with a diameter of 0.22 µm (#12).
   Enzyme reaction solution
      Solution A, 60 mL
      1000 units/mL DNase1 [1 × 10⁵ units/mL stock solution(^{∗}2)], 1.2 mL
      HBSS (#1), 58.8 mL
      The solution was prepared immediately before use.
      Besides, upon the reaction with an adipose layer, since the enzyme reaction solution is mixed with an equal amount of the adipose layer, the concentration of each enzyme reaction solution is set to be a half for the reaction.
*4: Preparation of fresh medium
   DMEM/Ham's F-12 (#15), 500 mL
   FBS (#16), 55 mL
   Penicillin-streptomycin (#17), 5 mL
*5: Preparation of fresh medium (not containing FBS)
   DMEM/Ham's F-12 (#15), 500 mL
   Penicillin-streptomycin (#17), 5 mL

### Example 1: Preparation of SVF

The sucked adipose tissues were left at rest, so that an oil layer, an adipose layer, and a water layer were separated from one another. The oil layer and the water layer were removed, and only the adipose layer was left. The weight of an empty centrifuge tube (#4) was measured. The size of the used centrifuge tube was set to be 500 mL with respect to 50 g of the adipose layer. The adipose layer was transferred into the centrifuge tube, followed by weighing the centrifuged tube. The weight of the adipose layer was calculated according the formula: (weight of centrifuge tube + adipose layer) - weight of centrifuge tube. The enzyme reaction solution (*3) was added in an equal amount to the adipose layer. The centrifuge tube was fixed in the constant temperature shaker (#6), followed by performing a reaction at 37°C at 120 rpm for 30 minutes. The tube was removed from the constant temperature shaker, and was then centrifuged using a centrifuge (#5) at 800 G for 10 minutes. After completion of the centrifugation, the resultant was separated into 3 layers, namely, an oil layer, an adipose layer, a water layer, and a precipitate layer (cell layer) from above. The oil layer, the adipose layer and the water layer were removed by pipetting, so that only the precipitate layer (cell layer) was left. Thereafter, HBSS (#1) was added to the precipitate layer, so that the amount of the HBSS became 90 mL with respect to 50 g of the adipose layer, and the obtained mixture was then washed by gentle suspending by pipetting. The obtained cell suspension was passed through 100 µm and 40 µm cell strainers to remove tissue sections. The resultant was centrifuged using a centrifuge (#5) at 800 G for 5 minutes. The supernatant was sucked, and was then re-suspended in 45 mL of HBSS (#1), and the obtained suspension was centrifuged at 700 G for 5 minutes. The supernatant was sucked, and was re-suspended in HBSS in an amount of 1 mL of HBSS with respect to 5 g of the adipose layer. The number of viable nucleated cells was counted using a cell counter (#10). SVF was suspended in the cryopreservation solution Cellbanker 1 (#13), and was then preserved at an extremely low temperature.

### Example 2: Passage culture of ASCs

Both in a state of waking up from a cryopreservation solution and in a fresh state, the stromal vascular fraction (SVF) was seeded in a concentration of 5.1 × 10⁴ viable nucleated cells/0.25 mL of medium (^{∗}4)/cm² in a culture flask (#14), and a culture was then initiated in a CO₂ incubator (#18) (37°C, 5% CO₂, Air). On the following day, the cells were washed with HBSS (#1) at 37°C three times, so that blood cells or remaining tissue sections were removed, and a fresh medium (*4) was then added. The medium was exchanged with a fresh medium once 3 days. On Day 7, in order to recover cells (mainly, ASCs), cell dispersion was carried out. The cells were washed with HBS (#1) at 37°C, and 1 x TrypLE express (#19) (1 mL TrypLE express/15cm²) at 37°C was added thereto, followed by performing cell dispersion at 37°C in 5% CO₂ for 5 minutes. The reaction was terminated with a fresh medium (*4), and the resulting cells were then centrifuged (300 G, 5 min, 4°C) to obtain cell pellets. The number of nucleated cells was counted using a cell counter (#10). The cells (mainly, ASCs) were seeded in a concentration of 4000 viable nucleated cells/0.25 mL of medium (^{∗}4)/cm² in a culture flask (#14), and a culture was then initiated in a CO₂ incubator (#18) (37°C, 5% CO₂, Air). The medium was exchanged with a fresh medium once three days, and the cells were then cultured for 7 days.

### Example 3: Inhibitory substances in CM

### < Outline >

Into CM, physiologically active substances derived from cells, which were expected to be effectiveness for tissue regeneration or cell culture, were released. At the same time, into such CM, metabolites inhibiting the survival and/or proliferation of cells were also released from the cells Thus, CM generally comprises both active ingredients and inhibitory substances. As a representative metabolite, lactic acid or ammonia that was a pure reagent was added, and the influence of the lactic acid or the ammonia on proliferation of ASCs was examined.

### < Procedure-1 >

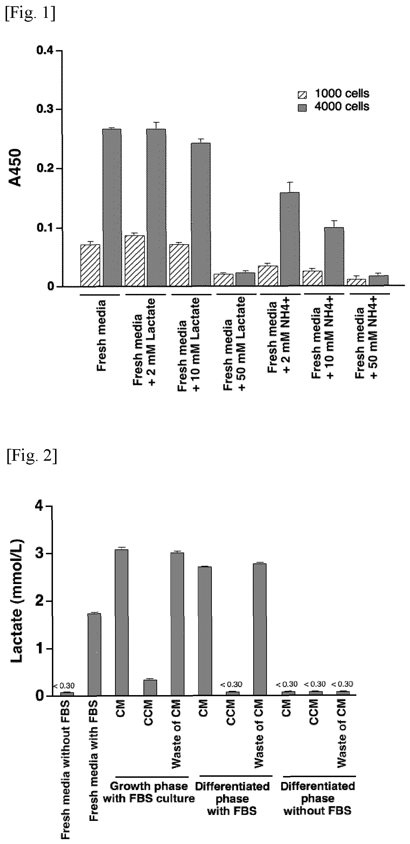

By the aforementioned SVF preparation in Example 1 and the aforementioned passage culture of ASCs in Example 2, ASCs were cultured up to a passage number (P) = 2, so as to obtain a cell suspension of the ASCs. The number of viable nucleated cells was counted using a cell counter (#10). The ASCs were seeded in a concentration of 4000 or 1000 viable nucleated cells/100 µL of medium (*4)/well on a 96-well plate (#22). Two of these well plates were prepared. A culture was initiated in a CO₂ incubator (#18) (37°C, 5% CO₂, Air). A fresh medium (*4), a fresh medium (*4) containing 2, 10, or 50 mM lactic acid (#20), and a fresh medium (*4) containing 2, 10, 50 mM ammonia (#21) were prepared, and 22 hours later, the medium was changed with a fresh one. Three days after initiation of the culture (2 days after the medium exchange), cell proliferation activity was confirmed using a cell counting kit (#23). The absorbance at 450 nm was measured using a plate reader (#24). The measurement results are shown in Fig. 1.

Compared with the fresh medium culture, in the medium into which lactic acid or ammonia had been added, inhibition of the cell proliferation activity occurred. In the case of lactic acid, culture inhibition was significantly observed in a 50 mM lactic acid-fresh medium. In the case of ammonia, culture inhibition of ASCs was observed in a 2 mM ammonia-fresh medium, and such culture inhibition significantly occurred in a 50 mM ammonia-fresh medium. The ammonia concentration in the culture supernatant (CM) recovered in the confluent phase was approximately 1.428 M NH₄+ (2000 µg/dL). Accordingly, it was found that the concentration of ammonia secreted from ASCs contained in CM is such a level that it inhibits cell proliferation, and that ordinary CM has properties by which the CM simultaneously comprises both physiologically active substances (beneficial substances) and metabolites (harmful substances).

### < Procedure-2 >

By the aforementioned SVF preparation in Example 1 and the aforementioned passage culture of ASCs in Example 2, ASCs were cultured up to a passage number (P) = 2, so as to obtain a cell suspension of the ASCs. The number of viable nucleated cells was counted using a cell counter (#10). The ASCs were seeded in a concentration of 4000 viable nucleated cells/0.2 µL of medium (^{∗}4)/ cm² on a cell culture dish (#14). CMs under the following conditions were recovered:
(Condition 1) CM obtained by exchanging the 60% to 70% confluent state medium with a fresh medium (*4) and then performing a culture for 72 hours,
(Condition 2) CM obtained by exchanging the 90% to 100% confluent state medium with a fresh medium (*4) and then performing a culture for 72 hours, and
(Condition 3) CM obtained by exchanging the 90% to 100% confluent state medium with a fresh medium (not containing FBS) (*5) and then performing a culture for 72 hours.

The CM was quickly recovered in a centrifuge tube, and was then centrifuged at 800 G for 10 minutes using a swing-type centrifuge, so that the cells and debris were precipitated in the CM and a supernatant was recovered. The supernatant was concentrated through an ultrafiltration membrane (#25) to obtain CCM-pre. A fresh medium (not containing FBS) (*5) was added to the CCM-pre concentrated on the ultrafiltration membrane to the maximum loading capacity (for dilution), and the CCM-pre was then concentrated again. By the aforementioned operations, from the CMs under the three culture conditions, CM, CCM obtained by substitution with a fresh medium and concentration (purification), and a waste liquid of CM as a flow-through upon ultrafiltration of the CM were each obtained. Lactic acid contained in each medium was quantified using a lactic acid quantification kit (#26). Besides, due to the properties of the measurement kit, a value of 0.3 mM or less of lactic acid is indicated to be < 0.30, and thus, such a low value cannot be measured as an actual value. The measurement results are shown in Fig. 2.

It became clear that, according to the present invention, as a result of the concentration using an ultrafiltration membrane and the substitution with a fresh medium and concentration, useful physiologically active substances can be concentrated, and at the same time, harmful low-molecular-weight metabolites can be eliminated.

### < Procedure-3 >

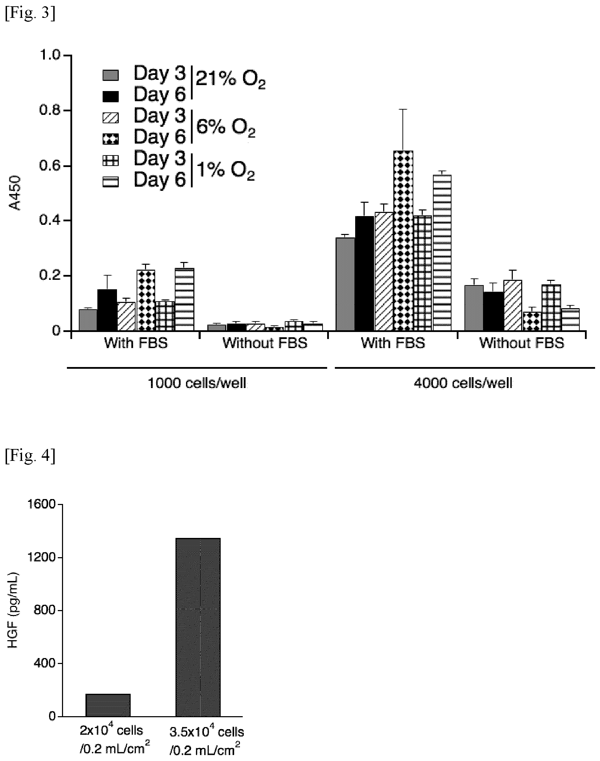

By the aforementioned SVF preparation in Example 1 and the aforementioned passage culture of ASCs in Example 2, ASCs were cultured up to a passage number (P) = 2, so as to obtain a cell suspension of the ASCs. The number of viable nucleated cells was counted using a cell counter (#10). The ASCs were seeded in a concentration of 4000 or 1000 viable nucleated cells/100 µL of medium (*4)/well on a 96-well plate (#22). A culture was initiated with 21% or 6% or 1% O₂ in a hypoxic CO₂ incubator (#27) (37°C, 5% CO₂). On Day 3 and Day 6 of the culture, cell proliferation activity was confirmed using a cell counting kit (#23). The absorbance at 450 nm was measured using a plate reader (#24). The measurement results are shown in Fig. 3.

With regard to the oxygen environment, proliferation efficiency was higher in the hypoxic condition (6% and 1%) than 21% O₂, and such proliferation efficiency was particularly favorable in 6% O₂. Considering the aforementioned results, CM was intended to be prepared under culture conditions of 37°C, 5% CO₂, and 6% O₂.

### Example 4: Preparation of CM

### < Outline >

In order to obtain CM in which large quantities of active ingredients were secreted, the optimal cell seeding density, medium amount, and culture period were studied in the case of culturing in an oxygen concentration (6% O₂) that was very similar to the physiological environment.

### < Procedure-1 >

By the aforementioned SVF preparation in Example 1 and the aforementioned passage culture of ASCs in Example 2, ASCs were cultured up to a passage number (P) = 1 (provided that the culture oxygen concentration was changed to 6%), so as to obtain a cell suspension of the ASCs. The number of viable nucleated cells was counted using a cell counter (#10). The cells were seeded on a cell culture dish (#14) under the following two conditions:
(Condition 1) ASCs were seeded in a concentration of 2 × 10⁴ viable nucleated cells/0.2 mL of medium (^{∗}4)/cm² (a seeding density by which ASCs became 90% confluent on the day following the seeding), and
(Condition 2) ASCs were seeded in a concentration of 3.5 × 10⁴ viable nucleated cells/0.2 mL of medium (^{∗}4)/cm² (a seeding density by which ASCs became 100% confluent on the day following the seeding).

On the following day, the medium was exchanged with an equal volume of fresh medium (*4), and 3 days after the medium exchange (4 days after initiation of the culture), the medium was quickly recovered in a centrifuge tube, and was then centrifuged at 800 G for 10 minutes using a swing-type centrifuge, so that the cells and debris were precipitated and a supernatant was recovered as CM. HGF that had been reported as a proliferation factor secreted by ASCs into the culture supernatant was quantified using Human HGF ELISA kit (#28). The measurement results are shown in Fig. 4.

Between Condition 1 (2 × 10⁴ ASC viable nucleated cells) (the seeding density by which ASCs became 90% confluent on the day following the seeding) and Condition 2 (3.5 × 10⁴ ASC viable nucleated cells) the seeding density by which ASCs became 100% confluent on the day following the seeding), there was a difference in terms of the amount of HGF in CM. From the above results, the ASC-seeding density and the amount of a medium were set to be Condition 2 (3.5 × 10⁴ ASC viable nucleated cells/0.2 mL of medium (^{∗}4)/cm²) in a 6% O₂ environment.

### < Procedure-2 >

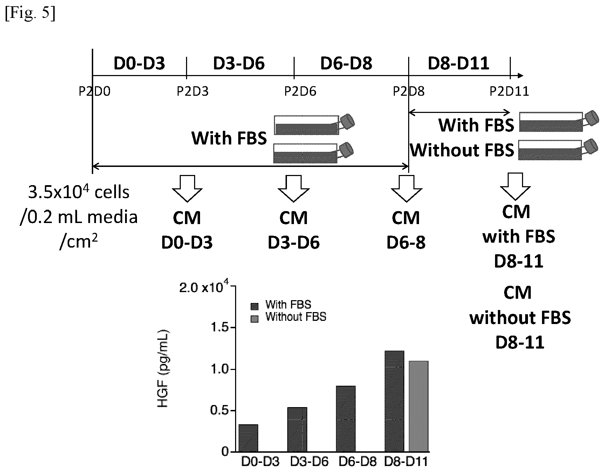

By the aforementioned SVF preparation in Example 1 and the aforementioned passage culture of ASCs in Example 2, ASCs were cultured up to a passage number (P) = 1, so as to obtain a cell suspension of the ASCs. The number of viable nucleated cells was counted using a cell counter (#10). The ASCs were seeded on a cell culture dish (#14) in a 6% O₂ environment under Condition 2 (3.5 × 10⁴ ASC viable nucleated cells/0.2 mL of medium (^{∗}4)/cm²). On Day 3, 6, 8, and 11, the culture supernatant was recovered, and an equal amount of fresh medium (*4) was then added thereto. Only on Day 11, the cells were cultured in a fresh medium group (*4) and in a fresh medium (not containing FBS) group (*5). The culture supernatant was quickly recovered in a centrifuge tube, and was then centrifuged at 800 G for 10 minutes using a swing-type centrifuge. The cells and debris were precipitated, and a supernatant was recovered as CM and was then preserved at -80°C. The amount of HGF was quantified using Human HGF ELISA kit (#28). The measurement results are shown in Fig. 5.

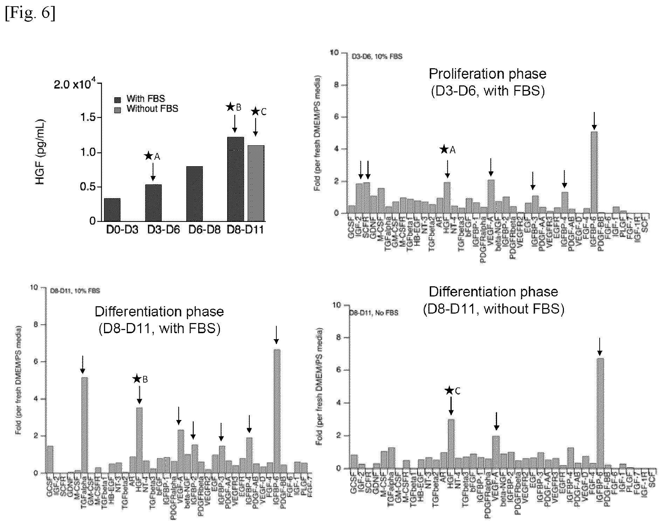

With regard to the following 4 conditions, namely, (1) D3-D6, (2) D8-D11 with FBS, (3) D8-D11 without FBS, and (4) a control fresh medium (not containing FBS) (*5), a semiquantitative analysis of secreted factors in CM was carried out with a growth factor antibody array (#29). Photographing was carried out using a lumino image analyzer (#30). The measurement results are shown in Fig. 6.

41 Types of proliferation factors comprised in the antibody array are as follows: Amphiregulin, bFGF, b-NGF, EGF, EGFR, FGF-4, FGF-6, FGF-7, G-CSF, GDNF, GM-CSF, HB-EGF, HGF, IGFBP-1, IGFBP-2, IGFBP-3, IGFBP-4, IGFBP-6, IGF-1, IGF-1sR, IGF-2, M-CSF, M-CSF R, NT-3, NT-4, PDGFRa, PDGFRb, PDGF-AA, PDGF-AB, PDGF-BB, PLGF, SCF, SCF R, TGF alpha, TGF beta 1, TGF beta 2, TGF beta 3, VEGF-A, VEGFR2, VEGFR3, and VEGF-D.

On the day following the seeding, even in the seeding density in which ASCs became 100% confluent, ASCs were further superimposed. The amount of HGF was increased in the second half of culture (differentiation phase) rather than in the first half of culture (proliferation phase) (Fig. 5).

Regarding the culture supernatant (CM) under each condition, it could be confirmed that the proliferation factor indicated with the arrow tended to increase (Fig. 6). Since the amount and type of a proliferation factor are different depending on the condition, CM can be recovered under the condition as needed. In addition, By purification and concentration using an ultrafiltration membrane, these proliferation factors are further concentrated.

The estimated values of the concentrations of individual cytokines in a concentrate, which are assumed by combining the measurement results of the antibody array and those of ELISA, are shown below.

When a culture supernatant is 30-fold concentrated, the concentrations of individual cytokines in the obtained concentrate are estimated as follows:
VEGF: 250 ng/mL,
FGF7 (KGF): 12000 pg/mL,
PDGF-AA: 50 ng/mL,
PDGFR-β: 125 ng/mL,
TGFβ: 1500 pg/mL,
IGFBP-6: 2000 ng/mL,
IGFBP-4: 350 ng/mL,
IGFBP-3: 170 ng/mL,
IGFBP-2: 270 ng/mL, and
IGFBP-1: 40 ng/mL.

### Example 5: Influence of oxygen concentration and presence or absence of serum (10% FBS) on HGF production

### < Measurement method/kit >

### ELISA method, Human HGF Quantikine ELISA Kit (R & D Systems, #DHG00B)

### < Measurement target sample >

### (1) Recovery of culture supernatant samples

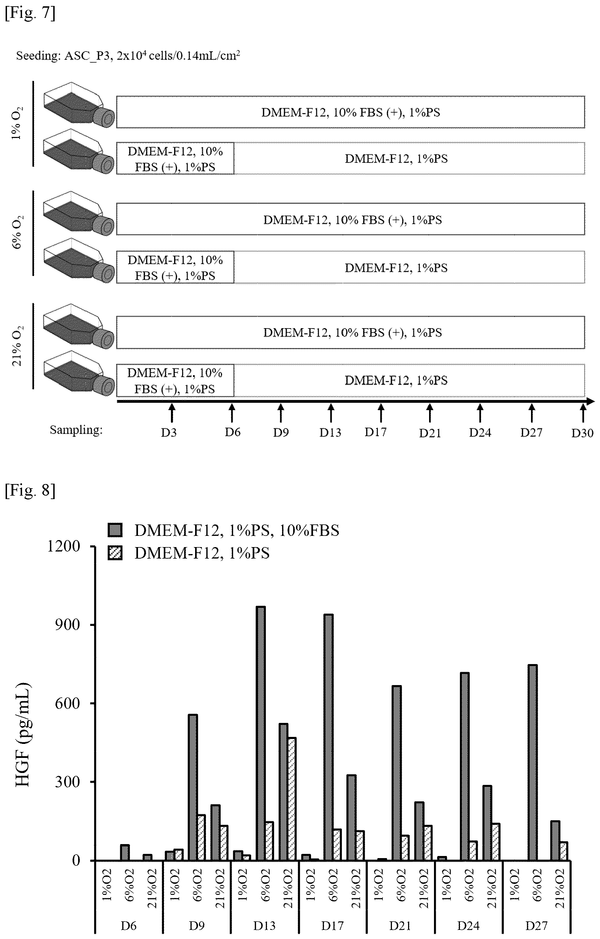

hASCs were seeded at an ordinary seeding density (2 × 10⁴ cells/cm²) in a flask, and were then cultured at 37°C in 5% CO₂, in an incubator in which the oxygen concentration was set at 1% O₂, 6% O₂, or 21% O₂. The schedule of the recovery of the culture supernatant and the medium exchange are shown in Fig. 7. In the medium exchange after the recovery on D6 (Day 6), both a serum (10% FBS)-containing medium and a serum-free medium were used properly.

### (2) Treatment of culture supernatant samples

From the recovered culture supernatant, cell debris and the like were removed by centrifugation, and a supernatant was preserved at -80°C.

### < Results >

The measurement results are shown in Fig. 8.

Regardless of the presence or absence of serum, only a very small amount of HGF was detected in the culture supernatant in the case of 1% O₂ environment culture. In contrast, under both 6% O₂ and 21% O₂ culture conditions, the amount of HGF produced was confirmed to be high in the culture with a medium containing 10% FBS, compared with a serum-free medium. Further, ASCs, which had been cultured in a medium containing 6% O₂ and serum, released a larger amount of HGF.

In an *in-vivo* environment, the oxygen concentration is said to be about 5% to 7% on average, and thus, when compared with the oxygen concentration in the atmosphere (ordinary oxygen environment), the *in-vivo* environment is a hypoxic environment. It has been considered that 6% O₂ closest to such *in-vivo* environment is more appropriate to the survival of ASCs, and thus that 6% O₂ is a condition favorable for production of HGF.

It is assumed that, in order to release a maximum amount of HGF into the culture supernatant, it would be preferable that large quantities of hASCs are seeded on a culture vessel, such that the adhesive surface of the culture vessel becomes confluent, and that a continuous culture is performed in a 10% FBS-added medium and under a 6% O₂ condition.

### Example 6: Quantitative measurement of HGF in culture supernatant, concentrate, and waste liquid after concentration

### < Measurement method/kit >

ELISA method, Human HGF Quantikine ELISA Kit (R & D Systems, #DHG00B)

### < Measurement target samples >

A culture supernatant, a concentrate, and a waste liquid after concentration, which were each prepared as a mixture of three times of cultures [cultured under a normal oxygen (21% O₂) condition)]

### < Methods >

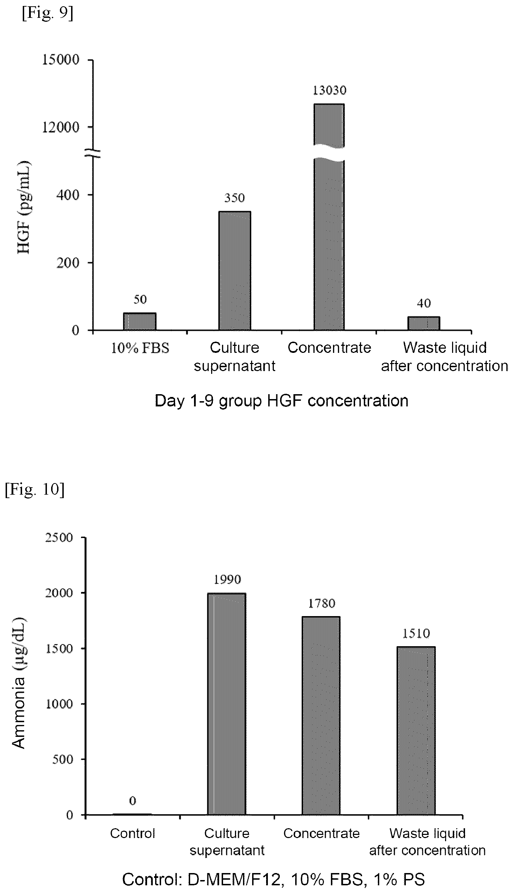

Useful factors (e.g. HGF) produced in the culture supernatant have a positive correlation with the number of hASC cells. Assuming that the HGF concentration is different depending on the recovery date, a recovery course for 1 month was divided into 3 groups, namely, Days 1 to 9, Days 10 to 18, and Days 19 to 30. Using a culture supernatant, a concentrate, and a waste liquid after concentration, which were each prepared as a mixture of three times of cultures, a quantitative analysis of HGF was carried out. The results obtained by performing a quantitative analysis on HGF contained in the culture supernatant, the concentrate, and the waste liquid after concentration, which were recovered on Days 1 to 9, are shown in Fig. 9.

### < Results >

In the culture supernatant, HGF secreted from the hASC cells was confirmed. The concentration of HGF in the concentrate was about 30 times higher than the concentration of HGF before concentration. Almost no HGF remained in the waste liquid after concentration.

### < Discussion >

It was assumed that the useful factors would increase in a concentrate obtained as a result of the filtration and concentration of the culture supernatant. This time, the hepatocyte growth factor (HGF) that was the most notable proliferation factor in the culture supernatant was used as a target for the analysis. In all of the recovery courses, the HGF concentration in the concentrate was 20 to 30 times higher than the HGF concentration in the culture supernatant before concentration.

### Example 7: Measurement of ammonia concentration in culture supernatant, concentrate, and waste liquid after concentration

### < Measurement method >

Fuji Drychem NX10N (FUJIFILM Medical Co., Ltd.)
Fuji Drychem Slide NH₃-PII (for plasma) (FUJIFILM Medical Co., Ltd.)

### < Measurement target samples >

A culture supernatant, a concentrate, and a waste liquid after concentration, which were each prepared as a mixture of 10 times of cultures (1 month), were used. As a control, D-MEM/Ham's F-12 (10% FBS, 1% PS) was used.

### < Results >

The quantifiable range of the ammonia concentration by Fuji Drychem Slide NH₃-PII (for plasma) is 10 to 500 µg/dL. Accordingly, each of the above-described samples was diluted so that the concentration thereof became in the quantifiable range, and was then measured. Thereafter, the measured value was further multiplied by a dilution multiple number, so that a precise measurement value could be calculated.

The measurement results are shown in Fig. 10.

### < Discussion >

From the above measurement results, no difference was found in the ammonia concentration among the culture supernatant, the concentrate, and the waste liquid after concentration. However, when the measurement values were converted to mass values, the amount of ammonia in the concentrate became about 1/30 of the amount of ammonia in the culture supernatant. As the number of operations of diluting the concentrate and performing ultrafiltration increases, the ammonia concentration in the liquid can be decreased to a measurement limit or less.

For example, the concentrate after completion of a first filtration is diluted (e.g. 30-fold dilution) by addition of a basal medium (sterilized distilled water, ultrapure water, a phosphate-buffered saline, PBS, etc.). The diluted solution is subjected to a second filtration using a stirring-type ultrafiltration device (e.g. 30-fold concentration). Thereby, regarding concentrates having a low molecular weight (in particular, harmful substances such as ammonia and lactic acid), the concentrates are almost replaced with the components of the basal medium, etc. Beside, since useful factors including a proliferation factor as a typical example have a high molecular weight, almost all of the factors are retained in the concentrate. Since these concentration and purification steps can be regulated in terms of the degree and the number of operations, the final concentration and purification degrees are infinite.

### Example 8: Influence of culture supernatant, concentrate, and waste liquid after concentration on proliferative ability of hASCs (cell proliferation experiment)

### < Measurement target samples >

Stock solutions of a culture supernatant, a concentrate, and a waste liquid after concentration, were each prepared as a mixture of 10 times of cultures (1 month).

A stock solution of the culture supernatant, the concentrate and serially diluted solutions of the concentrate, and the waste liquid after concentration and serially diluted solutions of the waste liquid after concentration were prepared as shown in Table 1 below. As a control, 1% PS-containing D-MEM/Ham's F-12 (hereinafter abbreviated as "DMEM-F12") was used, and as a vehicle group, DMEM-F12/10% FBS/1% PS was used.

**[Table 1]**

| Groups | Structural components |
|---|---|
| Vehicle | DMEM-F12/10% FBS/1% PS |
| Control | DMEM-F12/1% PS |
| CM | Stock solution of culture supernatant |
| CCM_1/100 | Solution 100-fold diluted from concentrate stock solution |
| CCM_1/30 | Solution 30-fold diluted from concentrate stock solution |
| CCM_1/10 | Solution 10-fold diluted from concentrate stock solution |
| CCM_1/3 | Solution 3-fold diluted from concentrate stock solution |
| CCM | Concentrate stock solution |
| W/CCM_1/100 | Solution 100-fold diluted from concentrate waste liquid |
| W/CCM_1/30 | Solution 30-fold diluted from concentrate waste liquid |
| W/CCM_1/10 | Solution 10-fold diluted from concentrate waste liquid |
| W/CCM_1/3 | Solution 3-fold diluted from concentrate waste liquid |
| W/CCM | Stock solution of concentrate waste liquid |

### < Methods >

In order to measure cell proliferative ability, Cell Counting Kit-8 (CCK-8, DOJINDO LABORATORIES , #CK04) was used. NADH generated by dehydrogenase in cells reduces water-soluble tetrazolium salt (WST-8) to orange-colored formazan via 1-methoxy PMS as an electron transfer substance. The amount of this formazan dye (maximum absorption wavelength: 450 nm) is proportional to the number of viable cells, and thus, it is possible to measure the number of viable cells by absorbance measurement. hASCs having a passage number of P4 were used, and were then seeded in a concentration of 3000 cells/well (96-well plate) (n = 4). The culture was carried out at 37°C in 5% CO₂ and in 21% O₂. The samples prepared as shown in Table 1 were each added in an amount of 100 µL/well onto the plate, and on Day 4 of the culture, a CCK-8 assay was carried out, and the absorbance (O.D. 450 nm) was measured using a microplate reader.

### < Results >

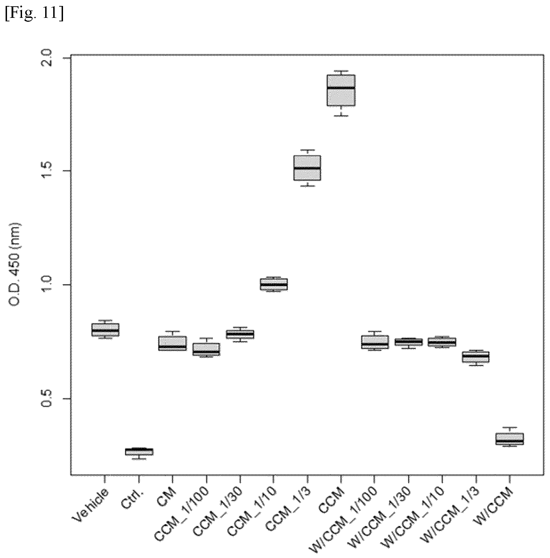

The measurement results are shown in Fig. 11.

Even the 100-fold diluted solution (CCM_1/100) of the concentrate significantly increased cell proliferation, compared with the control (DMEM-F12, 1% PS) group. This proliferation-promoting ability was not significantly different from the vehicle (DMEM-F12/10% FBS/1% PS). However, when the concentrate diluted solutions (10-fold diluted solution CCM_1/10 and 3-fold diluted solution CCM_1/3) were compared with the concentrate stock solution, the concentrate stock solution significantly promoted cell proliferation. When compared with the vehicle group, addition of the 3-fold diluted solution of the waste liquid after concentration (W/CCM_1/3) significantly suppressed cell proliferative ability, and the stock solution of the concentrate waste liquid (W/CCM) significantly reduced cell proliferative ability.

### Example 9: Influence of culture supernatant and detoxified secretome extract on proliferation of human dermal fibroblasts and normal human epidermal keratinocytes (cell proliferation experiment)

### < Reagents/Methods >

Normal human dermal fibroblasts (NHDFs) or normal human epidermal keratinocytes (NHEKs) were each seeded in a concentration of 1 × 10⁵cells/mL on a 96-well plate (n = 4). Twenty-four hours later, the medium was exchanged with a control or a serially diluted culture supernatant detoxified secretome extract.

In the case of NHDFs, D-MEM/Ham's F-12 as a control, or 5% FBS-added D-MEM/Ham's F-12 was used to prepare CCM (concentrate stock solution)-diluted solutions shown in Table 1. On the other hand, in the case of NHEKs, KGM-Gold (Lonza, #192060) medium was used as a control, and CCM (concentrate stock solution)-diluted solutions shown in Table 1 were prepared.

In order to measure cell proliferative ability, Cell Counting Kit-8 (CCK-8, DOJINDO LABORATORIES , #CK04) was used.

Seventy-two hours after the culture, a CCK-8 assay was carried out, and the absorbance (O.D. 450 nm) was measured using a microplate reader.

### < Results >

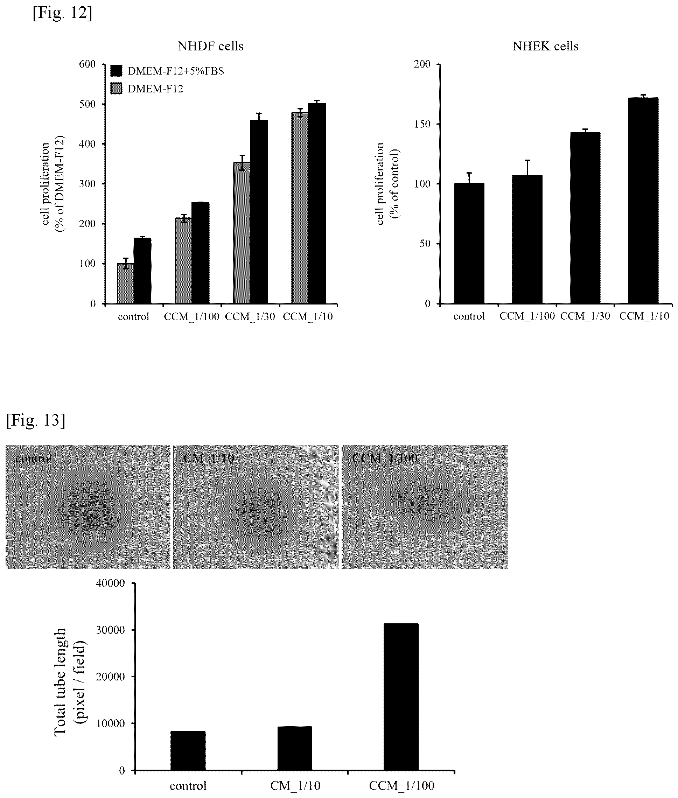

The measurement results are shown in Fig. 12.

It was confirmed that the cell proliferative ability of the human dermal fibroblasts was increased in a CCM concentration-dependent matter, regardless of the presence of 5% FBS (left view of Fig. 12).

Even in the normal human epidermal keratinocytes, it was found that cell proliferation was promoted by addition of CCM (right view of Fig. 12).

### Example 10: Influence of culture supernatant and culture supernatant detoxified secretome extract on tube-forming ability of vascular endothelial cells (endothelial cell tube formation test)

### < Measurement samples >

A culture supernatant (CM in Table 1) and a culture supernatant detoxified secretome extract (CCM in Table 1) were prepared as shown in the following table.

**[Table 2]**

| Groups | EBM2 | D-MEM/Ham's F-12 | CM | CCM |
|---|---|---|---|---|
| Control | 9 | 1 | | |
| CM_1/10 | 9 | | 1 | |
| CCM_1/100 | 9 | 0.9 | | 0.1 |

### < Methods >

In order to evaluate the tube-forming ability of endothelial cells, human umbilical vein endothelial cells (HUVECs) were used. HUVECs were cultured using an EGM-2 medium (Lonza, #CC-4176) until the cells entered the exponential growth phase. Before a tube formation analysis, HUVECs were left in a serum-free state for 12 hours. Matrigel Basal Membrane Matrix (Corning, #356231) was thawed at 4°C overnight, and it was always placed on ice during the experiment. The thawed Matrigel was added in an amount of 50 µL into each well of a 96-well plate, and was then left at rest in an incubator for 1 hour. Using TrypLE Express, the starving HUVECs were peeled, and were then pelletized. A control group and experiment groups were prepared as shown in the above table, and the HUVECs were re-suspended therein at a density of 1 × 10⁵ cells/mL. Into the wells containing Matrigel, 100 µL each of the cell suspension was added (the number of cells per well: 1 × 10⁴ cells). The plate was left under conditions of 37°C and 5% CO₂, and the cells were cultured for 4 to 6 hours. The cultured cells on the plate were photographed under a phase-contrast microscope (Leica) at 40-fold magnification. The level of the tube-forming ability was evaluated based on randomly photographed 5 microscopic images, by analyzing the length of the formed tube using the image analysis software ImageJ.

### < Results >

The measurement results are shown in Fig. 13.

When compared with the control group, a clear change was not found in the CM-diluted solution addition group. In the concentrate-diluted solution addition group, the action to promote tube formation was exhibited.

### Example 11:

### < Methods >

Eight-week-old db/db mice (BKS.Cg-+Lepr^{db}/+Lepr^{db}/J), and control mice (BKS.Cg-Dock7^{m}+/Dock7^{m}+/J) were used in the experiment. Each mouse was bred in a different cage, and thus, the mice were recognized individually. The dorsal portion of each mouse was shaved with chippers under ISOFLURANE inhalation, and the entire skin layer was peeled from the shaved portion using a sterilized biopsy punch (diameter: 6.0 mm, Kai industries), so as to create an ulcer. For the purpose of preventing wound contraction, a donut-type silicone splint (donut hole diameter: 9 mm, outer diameter: 15 mm, thickness: 1 mm, Kyowa Industrial Co., Ltd.) was fixed with Nylon 6-0. The date on which the ulcer was formed was defined to be Day 0, and on Day 0, Day 3 and Day 6, 0.1 ml each of the concentrate stock solution CCM shown in Table 1 was uniformly injected as a sample into the subcutis around the ulcer of each mouse, divided over 4 directions, using a syringe. The wound portion was dressed with Moiskin Pad, so that dissociation of the silicone splint and the drying of the wound portion were prevented. As CCM, a detoxified secretome extractd culture supernatant, which was obtained by culturing ASCs and then purifying and concentrating the culture, was used. The progress of the wound portion from Day 0 to Day 21 was macro-photographed with a single-lens reflex camera, and the wound healing-promoting effect was evaluated from the wound area. The mice were subjected to euthanasia by 5% ISOFLURANE inhalation on Day 21.

**[Table 3]**

| Group | Mice | Injection | Number |
|---|---|---|---|
| Control 1 (C1) | Control (-/-) | DMEM + 0.2% HA | 6 |
| Control 1 (C2) | db/db | DMEM + 0.2% HA | 7 |
| Experiment 1(E1) | db/db | CCM + 0.2% HA | 6 |
| Total | | | 19 |

### < Results >

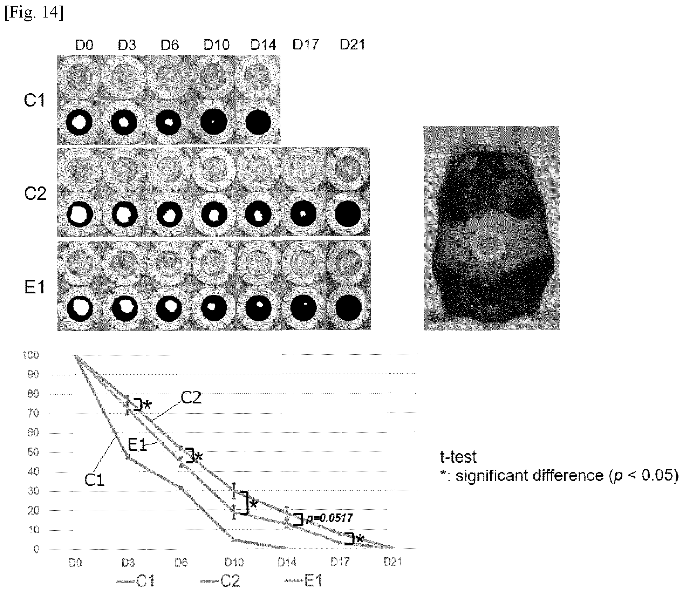

The evaluation results are shown in Fig. 14.

It became clear that the retard wound healing of type 2 diabetic mice was significantly improved by administration of CCM.

### Example 12: Influence of platelet lysate on cell proliferative ability and HGF-producing ability of ASCs

### < Reagents/Kits >

Platelet lysate (human platelet lysates, hPL)
Fetal bovine serum (FBS)
TrypLE Express (Gibco, #12604-021)
Human HGF Quantikine ELISA Kit (R & D Systems, #DHG00B)

### < Measurement target samples >

### (1) Recovery of culture supernatant samples

hASCs were seeded in a concentration of 2.5 × 10⁴ cells/mL on a 100-mm dish, and were then cultured at 37°C in a 5% CO₂ incubator. Twenty-four hours later, the medium was exchanged with D-MEM/Ham's F12 supplemented with each of 10% FBS and 5% hPL, and the cells were further cultured for 7 days.

### (2) Treatment of culture supernatant samples

After the cells had been cultured for 7 days, the culture supernatant was recovered from each dish, cell debris and the like were then eliminated by centrifugation, and the supernatant was preserved at -80°C. After the recovery of the culture supernatant, the cells were peeled using TrypLE Express, and the number of nucleated cells was then counted using a cell counter (Logos Biosystems, #L30001).

### < Results >

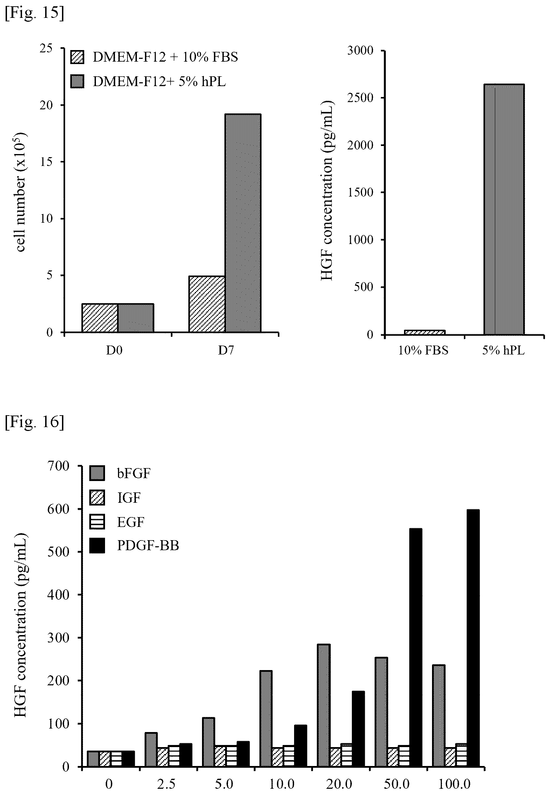

The measurement results are shown in Fig. 15.

As shown in the left view of Fig. 15, when the ASCs were cultured using the 10% FBS-added medium, the number of cells were about two times increased on D7, when compared with upon the seeding on Day 0 (D0). On the other hand, it was confirmed that, in the 5% hPL-added medium, the number of cells were about 8 times increased.

A comparison was made in terms of the HGF concentration in the culture supernatant recovered on D7, and as a result, it was confirmed that the amount of HGF released from hASCs cultured in 5% hPL was apparently large (right view of Figure 15).

### < Discussion >

It was confirmed that human platelet lysates (hPLs) enhanced the cell proliferative ability and HGF-producing ability of hASCs, rather than FBS.

### Example 13: Influence of various types of proliferation factors on HGF-producing ability of ASCs

### < Reagents/Kits >

Recombinant Human FGF basic/FGF2 (R & D Systems, #233-GMP)
Recombinant Human LR3 IGF-1/IGF-1 GMP (R & D Systems, #8335D-GMP)
Recombinant Human PDGF-BB (R & D Systems, #AF220)
Recombinant Human EGF (R & D Systems, #236-EG-200)
Human HGF Quantikine ELISA Kit (R & D Systems, #DHG00B)

< Measurement target samples >

### (1) Recovery of culture supernatant samples

hASCs were seeded in a concentration of 5 × 10⁴ cells/mL on a 48-well plate, and were then cultured at 37°C in 5% CO₂ and in 21% O₂. Twenty-four hours later, the medium was exchanged with D-MEM/Ham's F12 supplemented with each of bFGF (basic fibroblast growth factor), IGF (insulin-like growth factor), EGF (epidermal growth factor) and PDGF-BB (platelet-derived growth factor BB), and the cells were further cultured for 72 hours. The final concentrations of various types of proliferation factors in the hASCs were 0, 2.5, 5.0, 10.0, 20.0, 50.0, and 100.0 ng/mL.

### (2) Treatment of culture supernatant samples

After the cells had been cultured for 72 hours, the culture supernatant was recovered from each dish, cell debris and the like were then eliminated by centrifugation, and the supernatant was preserved at -80°C.

### < Results >

The results obtained by measuring the HGF-producing ability are shown in Fig. 16.

bFGF and PDGF-BB exhibited the action to promote the HGF production of the hASCs. The optimal concentration of bFGF was 20 ng/mL, and the optimal concentration of PDGF-BB was 100 ng/mL. Meanwhile, IGF and EGF did not influence on such HGF production.

### < Discussion >

Both of the factors bFGF and PDGF-BB promoted HGF production. bFGF exhibited the effects at a relatively low concentration, and such effects were exhibited until the final concentration of bFGF became 20 ng/mL. On the other hand, PDGF-BB promoted HGF production in a concentration-dependent manner.

PDGF-BB is a platelet-derived proliferation factor, and is contained in a large amount in a platelet lysates (PL). In culturing ASCs, it is considered that PL would be useful as a supplement, as well as FBS.

### Example 14: Influence of adjustment of stem cell culture method and purification and concentration steps on quality of detoxified secretome extract

### < Reagents/Kits >

Platelet lysates (human platelet lysates (hPL))
Human HGF Quantikine ELISA Kit (R & D Systems, #DHG00B)
Fuji Drychem NX10N (FUJIFILM Medical Co., Ltd.)
Fuji Drychem Slide NH₃-PII (for plasma) (FUJIFILM Medical Co., Ltd.)

### < Measurement target samples >

### (1) Recovery of culture supernatant samples

hASCs were seeded in a concentration of 2.5 × 10⁵ cells/mL on a 150-mm dish, and were then cultured at 37°C in a 5% CO2 incubator. As a medium, D-MEM/Ham's F12 supplemented with 5% hPL and specific supplements was used. Every 7 days, the recovery of a culture supernatant and medium exchange were carried out. Every recovery dates, lot numbers were assigned.

### (2) Ultrafiltration step of culture supernatants

Cell debris and the like were eliminated from the recovered culture supernatant by centrifugation, and the supernatant was subjected to a first filtration using a stirring-type ultrafiltration device, so that the supernatant was about 30-fold concentrated. Thereafter, the "first concentrate" obtained after the first filtration was 30-fold diluted with a phosphate buffered saline (PBS), and was then subjected to ultrafiltration again. Accordingly, a second ultrafiltration was performed on 340 mL of the culture supernatant with each different lot number recovered in the above (1), so as to obtain about 10 mL of a detoxified secretome extract. Using the detoxified secretome extract obtained after completion of the second filtration as a subject, the HGF concentration and the ammonia concentration in the detoxified secretome extract were measured.

### < Measurement methods >

The HGF concentration was measured using Human HGF Quantikine ELISA Kit. The ammonia concentration was measured using Fuji Drychem NX10N and Fuji Drychem Slide NH₃-PII (for plasma).

### < Measurement results >

The measurement results are shown in Table 4 below.

**[Table 4]**

| Lot # | Ammonia (µg/dL) | HGF (pg/mL) |
|---|---|---|
| B21176J | 9.3 | 398,547.0 |
| B21202J | 8.7 | 437,734.9 |
| B21226J | 7.3 | 456,603.8 |
| B21203J | 9.0 | 122,040.0 |

### < Discussion >

By controlling the culture method (supplements added to the basal medium) and the number of ultrafiltration operations, a detoxified secretome extract having a high HGF concentration and a low ammonia concentration could be obtained.

### Example 15: Influence of culture supernatant and detoxified secretome extract on inflammatory cytokine production

### < Reagents/Kits >

Human IL-6 Quantikine ELISA Kit (R & D Systems, #D6050)
Lipopolysaccharides from Escherichia *coli* O55:B5 (Sigma-Aldrich, #L6529)

Culture supernatants and detoxified secretome extracts were prepared as shown in the following table.

**[Table 5]**

| Groups | Structural components |
|---|---|
| Control | DMEM-F12 |
| Vehicle | DMEM-F12/10% FBS |
| CM | Stock solution of culture supernatant |
| CCM_1/100 | Solution 100-fold diluted from concentrate stock solution |
| CCM_1/30 | Solution 30-fold diluted from concentrate stock solution |

### < Measurement target samples >

### (1) Recovery of normal human dermal fibroblast-acclimatized culture solution samples

Normal human dermal fibroblasts (NHDFs) were seeded in a concentration of 3 × 10⁵ cells/mL on a 6-well plate (two plates), and were then cultured at 37°C in 5% CO₂ and in 21% O₂. Twenty-four hours later, the medium was exchanged with D-MEM/Ham's F12, and the cells were left in a serum-free state for 6 hours. The medium for starving NHDFs was exchanged with the medium prepared in Table 5. Twenty-four hours later, the medium was removed with an aspirator, and the NHDF cells were then washed twice with HBSS (Hanks' Balanced Salt Solution). Thereafter, the resulting NHDF cells were cultured for 6 hours in a D-MEM/Ham's F12 medium, which was not supplemented with lipopolysaccharides (LPS) or was supplemented with LPS having a final concentration of 100 ng/mL. Six hours later, NHDF cell-acclimatized culture solutions under individual concentrations were each recovered.

### (2) Treatment of NHDF cell-acclimatized culture solution samples

Cell debris and the like were eliminated from the NHDF cell-acclimatized culture solutions recovered in the above (1) by centrifugation, and the supernatants were then preserved at -80°C.

### < Measurement method >

The concentration of the inflammatory cytokine IL-6 in the NHDF cell-acclimatized culture solution was measured by an ELISA method.

### < Results >

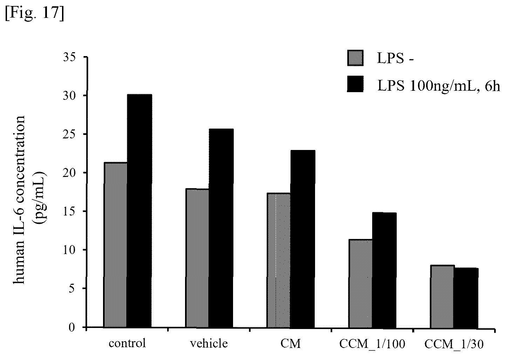

The measurement results are shown in Fig. 17.

It was demonstrated that addition of 100 ng/mL LPS to the NHDF cells promoted the secretion of IL-6 as an inflammatory cytokine. In addition, it was also demonstrated that the IL-6 secreting ability of the NHDF cells was suppressed in the culture supernatant stock solution (CM) addition group, compared with the control group, regardless of the presence of LPS, and that IL-6 production was significantly suppressed in the 100-fold diluted solution (CCM_1/100) of the concentrate stock solution addition group and in the 30-fold diluted solution (CCM_1/30) of the concentrate stock solution addition group.

### < Discussion >

It has been known that IL-6 is a cytokine playing an important role in regulation of immune response or inflammatory reaction, and that excessive production of IL-6 causes various pathological conditions. A culture supernatant of stem cells exhibited a slight anti-inflammatory action, but a culture supernatant concentrate thereof exhibited an apparent anti-inflammatory action although the additive amount thereof was small. It has been suggested that the culture supernatant concentrate should be applied as an anti-inflammatory agent.

## Claims

1. A detoxified secretome extract of a culture supernatant of mesenchymal stem cells or progenitor cells derived from the mesenchymal stem cells, wherein
the detoxified secretome extract comprises at least one of IGFBP, HGF, VEGF, PDGF, EGF, KGF (FGF-7), PDGFR, TGFα, and TGFβ secreted by the mesenchymal stem cells or the progenitor cells derived from the mesenchymal stem cells, and
lactic acid and ammonia that are metabolic waste products caused by the mesenchymal stem cells or the progenitor cells derived from the mesenchymal stem cells are removed from the detoxified secretome extract.

2. The detoxified secretome extract according to claim 1, which is used as a therapeutic agent for regenerative medicine, an immunosuppressant, an anti-inflammatory agent, or an antifibrotic agent.

3. The detoxified secretome extract according to claim 1 or 2, which comprises all of IGFBP, HGF, VEGF, PDGF, EGF, KGF (FGF-7), PDGFR, TGFα, and TGFβ secreted by the mesenchymal stem cells or the progenitor cells derived from the mesenchymal stem cells.

4. The detoxified secretome extract according to any one of claims 1 to 3, wherein at least one of IGFBP, HGF, VEGF, PDGF, EGF, KGF (FGF-7), PDGFR, TGFα, and TGFβ is 1.2 times or more concentrated, compared with the culture supernatant before concentration.

5. The detoxified secretome extract according to any one of claims 1 to 4, wherein the mesenchymal stem cells or the progenitor cells derived from the mesenchymal stem cells are adipose-derived stromal/stem cells or adipose-derived vascular endothelial progenitor cells.

6. The detoxified secretome extract according to any one of claims 1 to 5, wherein the ammonia concentration is 20 µg/dL or less, and the HGF concentration is 50000 pg/mL or more.

7. A method for producing the detoxified secretome extract according to any one of claims 1 to 6, comprising:
a culture supernatant-obtaining step of obtaining a culture supernatant by culturing mesenchymal stem cells or progenitor cells derived from the mesenchymal stem cells in a culture solution;
a concentration step of concentrating an active ingredient from the culture supernatant; and
a purification step of removing metabolic waste products from the concentrated culture supernatant.

8. The method according to claim 7, wherein, in the culture supernatant-obtaining step, a culture supernatant is obtained from a culture of the mesenchymal stem cells that are in a proliferation phase or a confluent phase, or the progenitor cells derived from the mesenchymal stem cells.

9. The method according to claim 7 or 8, wherein the metabolic waste products removed in the purification step are lactic acid and ammonia.

10. The method according to any one of claims 7 to 9, wherein the purification step is carried out by ultrafiltration.

11. The method according to claim 10, wherein the ultrafiltration is carried out using a filtration membrane having a cutoff molecular weight of 2 kDa to 30 kDa.
